# EUROPEAN PATENT SPECIFICATION

(11) **EP 2 338 122 B1**
(45) Date of publication and mention of the grant of the patent: **05.12.2018**
(21) Application number: 09787154.5
(22) Date of filing: 09.09.2009
(51) Int. Cl.: G16H 50/70, G16H 50/20

(54) **SYSTEM AND METHOD FOR FUSING CLINICAL AND IMAGE FEATURES FOR COMPUTER-AIDED DIAGNOSIS**
SYSTEM UND VERFAHREN ZUR MEDIZINISCHEN DIAGNOSE MITTELS EINER FUSION VON KLINISCHEN UND BILD MERKMALEN
SYSTÈME ET PROCÉDÉ DE FUSION DE CARACTÉRISTIQUES CLINIQUES ET D'IMAGE POUR UN DIAGNOSTIC ASSISTÉ PAR ORDINATEUR (CAD)

(30) Priority: 26.09.2008 US 100307 P
(43) Date of publication of application: 29.06.2011
(73) Proprietor: Koninklijke Philips N.V., 5656 AE Eindhoven (NL)
(72) Inventor: LEE, Michael, Chun-chieh, Cleveland Ohio 44143 (US); BOROCZKY, Lilla, Cleveland Ohio 44143 (US)
(74) Representative: van Velzen, Maaike Mathilde
(86) International application number: PCT/IB2009/053950
(87) International publication number: WO 2010/035161

(56) References cited:
- US-A1- 2004 193 036
- US-A1- 2006 034 508
- US-A1- 2006 200 010
- US-A1- 2008 171 916
- US-B2- 7 244 230
- MCSHERRY D: "Avoiding premature closure in sequential diagnosis" ARTIFICIAL INTELLIGENCE IN MEDICINE, ELSEVIER, NL, vol. 10, 1 January 1997 (1997-01-01), pages 269-283, XP002314882 ISSN: 0933-3657

## Description

### DESCRIPTION

The present application relates to the art of medical diagnosis. It finds particular application to computer-aided diagnosis (CADx) algorithms, and pattern classification algorithms However, it will also find application in other fields in which medical diagnosis is of interest.

One type of a CADx system can estimate the likelihood of a malignancy of a pulmonary nodule found on a CT scan. However, unlike computer-aided detection algorithms that rely solely on image information to localize potential abnormalities, the decision-making process associated with evaluation of malignancy typically includes integration of non-imaging evidence. Analysis of a CT scan image alone is rarely sufficient for assessment of a solitary pulmonary nodule. Critical studies have demonstrated that both diagnostic ratings and perception of radiological features are affected by patient histories. Specifically for lung nodules, studies have explicitly analyzed the degree to which clinical risk factors modulate the statistical probability of malignancy. The development of computer-aided diagnosis algorithms has therefore included clinical features to supplement the information in images.

Integrating different data types such as, but not limited to, clinical and imaging data, has a direct relevance to the way in which algorithms are accessed by a user and the workflow that is engaged when using the system. For efficiency of performance reasons, it is desirable to perform as much of the computer-aided diagnosis computation as possible before the user accesses the system. One problem with current diagnostic systems is they are inefficient because current systems require all data to be entered, irrespective of whether the data is actually necessary to make a diagnosis. It is therefore desirable to minimize the amount of information that the user has to enter, such as for example, by minimizing or eliminating entry of extraneous clinical data that will not significantly change the diagnosis. Clinical information can be drawn from an electronic health record. However, data fields may be missing or incomplete and information may be unknown. Another problem with current diagnostic systems is that they lack a technique for handling
missing or incomplete clinical information. So, it is desirable to develop a calculation that can assess and present the range of possible outcomes within the clinical information that is available.

The present application provides an improved system and method which overcomes the above-referenced problems and others.

The invention is defined by the claims.

According to one aspect of the invention, there is provided a system for providing computer-aided analysis of medical images comprising:
an image processor for processing medical image data;
a decision engine for generating a diagnosis based on the processed medical image data alone and further computing possible diagnostic outcomes based on different proposed possible values for clinical data;
a database of prior diagnoses, their accompanying probabilities, and classifier algorithms for assessing probability of an illness given image data alone, image data with incomplete clinical data, and/or image data with clinical data;
an interface engine for requesting and entering clinical data; and
a display terminal for displaying the results of the computer-aided analysis,
wherein the system is further adapted to develop ensembles of classifiers for use in the computer aided analysis, the developing comprising
making available a set of patient cases, each with multiple data types, as a training database for training,
inducing a decision tree on the clinical data as a first level classifier to roughly classify the patients based on a final outcome,
using the decision tree to stratify the patients in the training data base into high risk or low risk of having a particular illness,
developing a set of possible classifiers based on the clinical data, ignoring any stratification of the patients,
performing a test for each of the classifiers for performance on the training data, the classifiers with high performance on each strata of patients being kept in separate groups such that the result for y strata of the patients includes y sets of the classifiers,
placing the z best performing classifiers on each strata or all classifiers with a minimum required performance of accuracy, sensitivity, or specificity, in the corresponding classifier set, the set of classifiers for each strata forming a classifier ensemble, and
storing the clinical decision tree and separate classifier ensembles as output.

In described examples, a system is presented for performing a computer-aided diagnosis using medical images data. The system makes a medical diagnosis by comparing medical records and probabilities in a database with the current image data to hypothesize a medical diagnosis and present a probability that the diagnosis is correct. Should the probability of the diagnosis fall below a threshold level, the system prompts the medical user to enter further clinical data in order to provide more information upon which the system can produce a medical diagnosis with a higher probability of being correct.

In further described examples, a method is presented for performing a computer-aided diagnosis using medical images. The method entails performing a medical diagnosis by comparing medical records and probabilities in a database with the current image data to hypothesize a medical diagnosis and calculate a probability that the diagnosis is correct. Should the probability of the diagnosis fall below a certain threshold level, the method then calls for the medical user to obtain further clinical data in order to provide a larger basis of information upon which a more accurate and more certain medical diagnosis may be performed.

A further advantage in some examples is improved efficiency for breaking the computation into smaller components for workflow improvements. Not all of the data is retrieved until such time as the data is necessary. Data need not be retrieved until the data is deemed necessary for the patient.

A further advantage is provided in some examples for handling of missing or incomplete clinical information.

A still further advantage is providing in some examples an interface and system workflow that splits the CADx calculation into two or more steps, based on the availability of data.

A still further advantage is providing in some examples a computational method for integrating the different data streams as they become available.

Still further advantages and benefits will become apparent to those of ordinary skill in the art upon reading and understanding the following detailed description.

The present application may take form in various components and arrangements of components, and in various steps and arrangements of steps. The drawings are only for purposes of illustrating the preferred embodiments and are not to be construed as limiting the present application.
FIGURE 1A illustrates a CADx diagnostic method;
FIGURE 1B illustrates a CADx diagnostic system;
FIGURE 2 illustrates an approach for creation of an algorithm for classification;
FIGURE 3 illustrates a training methodology for the classifier;
FIGURE 4 illustrates another approach for creation of an algorithm for classification;
FIGURE 5 illustrates another classifier that incorporates an ensemble;
FIGURE 6 a illustrates the manner in which a classifier works with a new and unknown case;
FIGURE 7 illustrates the manner in which Bayesian analysis performs risk analysis;
FIGURE 8 illustrates proof-of-concept experimental results; and
FIGURE 9 illustrates the layout of the system and interaction between components.

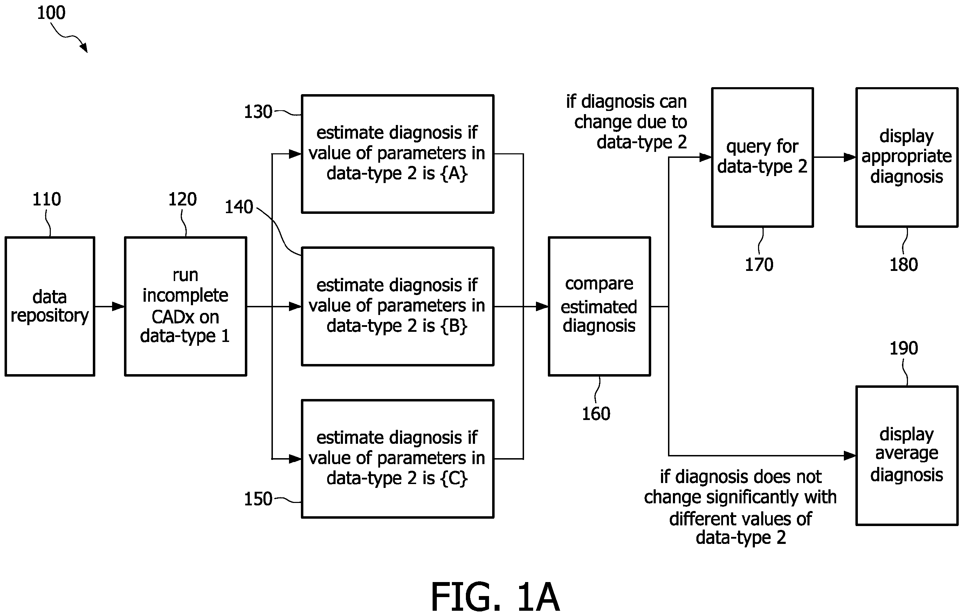

With reference to FIGURE 1A, a computer-aided diagnosis method 100 includes a CADx classifier algorithm that optimally runs on two types of data ('data-type 1' and 'data-type 2'). Clinical data describes aspects of the patient's health history, family history, physique, and lifestyle, including such elements as, but not limited to, smoking, previous illnesses, and the like. Image data comprises x-rays, CT scans, and any other type of medical imaging performed on a patient. The CADx algorithm combines image data in CT images (data-type 1, in this example) with clinical data in the clinical parameters (considered data-type 2 in this example) of the patient (e.g. emphysema status, lymph node status).

The first step in the method comprises a step of retrieving a set of data associated with a patient from a data repository 110. This data may include one or more quantitative variables. Data-type 1 is retrieved instead of data-type 2 if for example data-type 1 is more readily available, as is the case in the present example. This retrieval preferably occurs without user interaction. For example: A CT volume of a thoracic scan (data-type 1 in this example) is retrieved automatically from a hospital PACS (Picture Archiving and Communication System).

The next step comprises applying a CADx algorithm 120 to the data-type 1 data. The result of this calculation does not yet represent the final diagnosis of the CADx algorithm step. This would preferably occur without user interaction. For example: The CADx step 100 runs a computer-aided detection algorithm to localize a lung nodule on the scan, runs a segmentation algorithm, to define the boundaries of the lung nodule, processes an image to extract from the image data a set of numerical features describing the nodule. A pattern classification algorithm, then estimates the likelihood that this nodule is malignant, based solely on the imaging data.

The method 100 has not yet received the data-type 2 data to complete the diagnosis. The method 100 therefore tests different proposed possible values of data-type 2 data (in this case, three different possible values, represented by three different arrows), completing the CADx calculation using these test values through operations performed by operation steps 130, 140, 150. If N different values of data-type 2 are possible, then N CADx results are computed, one for each test value of data-type 2. For example: The CADx algorithm adjusts the image-based classification output based on all the different proposed possible combinations of emphysema and lymph node status. Since these are both binary variables (yes/no), four different combinations are possible. As a result, the CADx now has four potential solutions for the likelihood of malignancy. This step becomes more complicated if the number of possible values is very large, or if some of the variables are continuous. These outputs are consolidated as output by a computer operable software means and used as input to a comparator.

A computer operable software means comparator step 160 compares the N different candidate CADx calculation results or potential solutions for the likelihood of malignancy and decides if they are within a pre-set tolerance. The tolerance can be set before the product is deployed in the field, or can be set by the user. If the candidate CADx results are within the pre-set tolerance (i.e. knowing data-type 2 makes no difference, so data-type 1 was sufficient to create a diagnosis) then a display step 190 displays for the user one or more of the following: the mean, median, range, or variance of the CADx calculation results. The results may be displayed graphically. For example: for one patient, the CADx algorithm finds that the four combinations of emphysema and lymph node status yield likelihoods of malignancy of 0.81, 0.83, 0.82, and 0.82, on a scale of 0-1. Since these are all very close in value, there is no need to ask the user for these variables or query a second database. When the radiologist loads the case, the method has already completed all preceding steps and reports that the CADx algorithm estimates a likelihood of malignancy of between 0.81-0.83.

If the candidate CADx calculation results are too different (i.e. knowing data-type 2 could change the diagnosis, and so it is important to gather that information), then the method requires 170 the user to present the significant clinical information. This exact information is then used to identify which of the N CADx output values to display 180 to the user. For example: for a different patient, the CADx method finds that the four combinations of emphysema and lymph node status yield likelihoods of malignancy of 0.45, 0.65, 0.71, and 0.53, on a scale of 0-1. The four estimates are so different that data-type 2 could change the diagnosis. When the radiologist loads the case, the method has already completed all preceding steps but reports to the radiologist that additional information (i.e., data-type 2) is needed to complete the CADx calculation. Emphysema and lymph node status are input manually by the user. Based on the added type 2 data, the CADx selects one of the four likelihoods (e.g. 0.65) as its final estimate. This final result is displayed 180 to the user.

If the additional data-type 2 data is requested and is not available, then the N possible results can then be presented to the user with the disclaimer that there is insufficient data to complete the calculation. For example: for a different patient, the lymph node status is not available, perhaps because the scan did not cover the necessary anatomy. The radiologist therefore enters the correct emphysema status but reports the lymph node status as unknown. Using the emphysema data, the computer is able to narrow the range of possible outputs from (0.45, 0.65, 0.71, 0.53) down to (0.45, 0.53), but is still unable to predict whether the nodule is more likely (>0.50) malignant or more likely not malignant (<0.50). The method thus reports to the radiologist that the estimate for the patient's likelihood of cancer is 0.45-0.53, but additional data would be required to further narrow the solution. This process can be extended in a hierarchical manner, appending additional data streams, each with additional test values and candidate solutions.

The algorithm within the CADx method described above can be used to perform the underlying calculation. The initial data-type 1 data calculation may extract images, but is not a classification step. However, the number of clinical features is large, and the variety of potential values makes an exhaustive testing of all possible combinations impractical. Therefore, novel approaches are used to fuse the clinical and imaging features in a way that directly parallels the workflow described above. The description of the methods are given in terms of a lung CADx application example and assuming data-type 1 is imaging data and data-type 2 is clinical data. However, the method should be considered general to any CADx classification task requiring multiple data streams.

Three different algorithmic approaches to split the data produced by the CADx into parts are presented herein: (A) classifier selection Approach I; (B) classifier selection Approach II; (C) Bayesian analysis.

A method in which categorical clinical data are converted into a numerical form compatible with the image data. The transformed clinical data are then treated equivalently with respect to the image data during data selection and classifier training. An example of such a transformation is a 1-of-C encoding scheme. After this encoding, no differentiation is made between data derived from the imaging data or the encoded categorical clinical variables. The lung CADx application presents a new method for performing this data fusion.

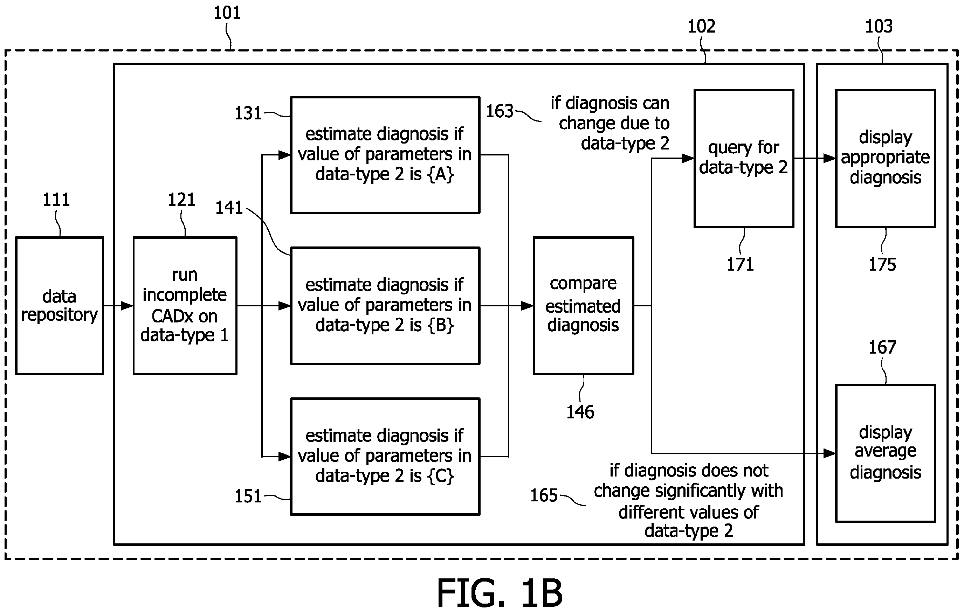

With reference to FIGURE 1B, a system 101 for fusing the clinical and image in the computer-aided diagnosis method 100 is presented, which incorporates a computer operable apparatus including, but not limited to a computer database data storage embedded within a computer memory, a computer output display terminal, a keyboard for inputting data, an interface for the import and extraction of data and any hardware and software components needed to make the proposed application function. The system performs the steps of the method 100 described in Figure 1. The system uses software which processes the data from the data repository 111. The software is run on a processor 102 which implements the incomplete data on a CADx algorithm 121 based system. The data is processed using a processor 102 which includes software that performs at least one of three estimates 131, 141, 151, and then moves this created data to a comparitor 146. The comparator uses computer operable computational means to evaluate whether the diagnosis based on incomplete data is significantly different than estimated diagnoses created with complete data. If the incomplete data and the completed data diagnoses data do not differ significantly 165, then the two results are an average of the two results are presented 167 by the processor 102 and displayed on a computer output means 103, such as a video display. However, if the results are different 163, then a query is performed for data type 2 data 171 and a diagnosis is presented by the processor 102 and displayed 175 in a computer operable output means 103.

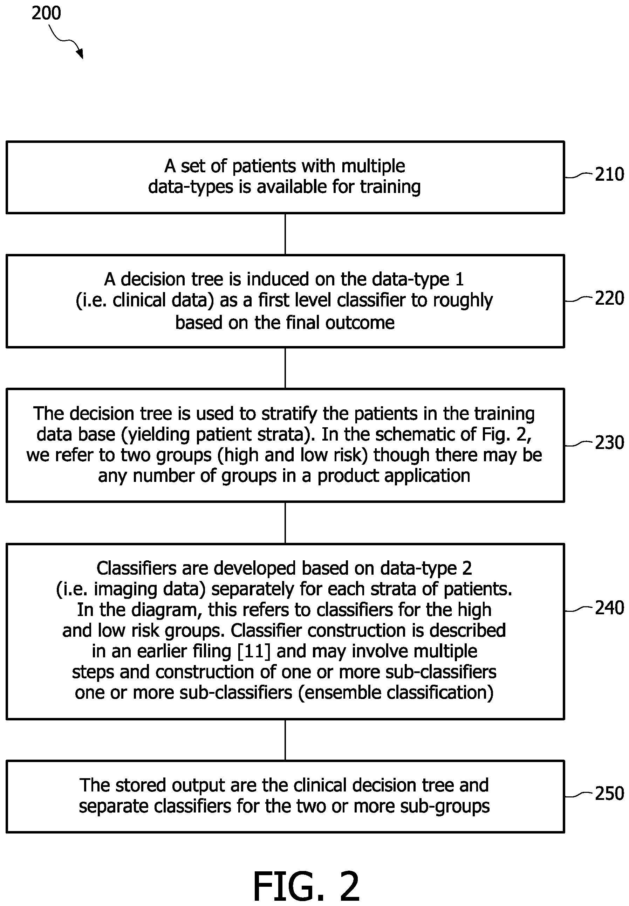

With reference to FIGURE 2, a first classifier selection method 200 (Approach I) is based on creating specific classifier(s) for different sub-groups of patients. The method of developing such an algorithm begins with step 210, wherein a set of patients with multiple data-types are made available for training. In the next step 220, a decision tree is induced on the data-type 1 image data as a first level classifier to roughly classify the patients based on the final outcome. The decision tree is then used in step 230 to stratify the patients in the training data base, also known as yielding patient strata. The Figure 2 schematic 200 refers to two groups high risk and low risk though there may be any number of groups in a product application. Classifiers are then developed in step 240 based on data-type 2 clinical data separately for each strata of patients. In the diagram, this refers to classifiers for the high and low risk groups. Classifier construction may involve multiple steps and construction of one or more sub-classifiers ensemble classification. In step 250, the clinical decision tree and separate classifiers for the two or more sub-groups are stored output.

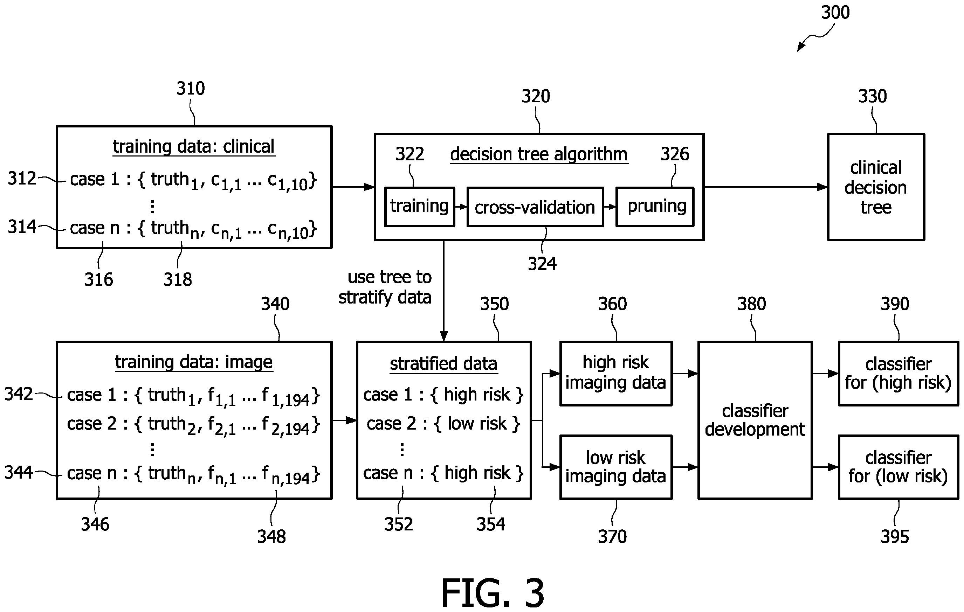

With reference to FIGURE 3, a training methodology for classifier selection has a goal of creating specific image-based classifiers for different clinical 'risk' groups. The diagram in Fig. 3 shows how the clinical and image data are combined to perform a diagnosis 300. The clinical data 310 is a collection of cases beginning with a first case 312 and proceeding to a given N cases 314, where N is an integer representing the number of cases, with each individual case representing a particular patient. Each case contains a name or identifier 316 of a patient and a series of attributes 318 gathered about the patient. These attributes include but are not limited to smoking, and exercise, or physical attributes such as but not limited to height and weight. These attributes also necessarily include the truth associated with the diagnosis in question, such as but not limited to whether the patient has cancer. These are input into the decision tree algorithm 320, which includes modules for training 322 for the creation of new decision tree branches, cross validation means 324 for checking of branches, and pruning means 326 for removing branches that are no longer relevant. The decision tree algorithm 320 is used to produce or output the clinical decision tree 330.

The training data for images 340 includes a series of cases beginning with a first case 342 and proceeding to a given N number of cases 344, with each individual case representing a particular patient. The cases 342, 344 represent the same patients as cases 312, 314. Each case contains a name or identification 346 of a patient and a series of attributes 348 gathered about the patient and the medical images of the patient. The attributes necessarily include the truth associated with the diagnosis in question, such as but not limited to whether the patient has cancer. The attributes further include but are not limited to descriptive features of the images and regions of the images, such as but not limited to descriptors of contrast, texture, shape, intensity, and variations of intensity. These cases from the training data for images 340 are used in combination with the decision tree algorithm 320 and clinical data 310 to create stratified data 350.

The stratified data 350 is generated to determine if an individual case presents a high risk 352 or a low risk 354 of possessing a given illness or condition based on whether the probability of a person with a specific health background is likely to have or not have a given illness or condition, i.e. based on the information contained within the clinical data 310. A person with a high likelihood is classified as high risk 360 imaging data, while a person with a low likelihood of such an illness are classified as low risk 370. Both high risk 360 and low risk 370 persons are analyzed by the classifier development means 380. A specific image classifier 390 is developed by means of 380 and input training data 360 to classify high risk patients. A specific image classifier 395 is developed by means of 380 and input training data 370 to classify low risk patients.

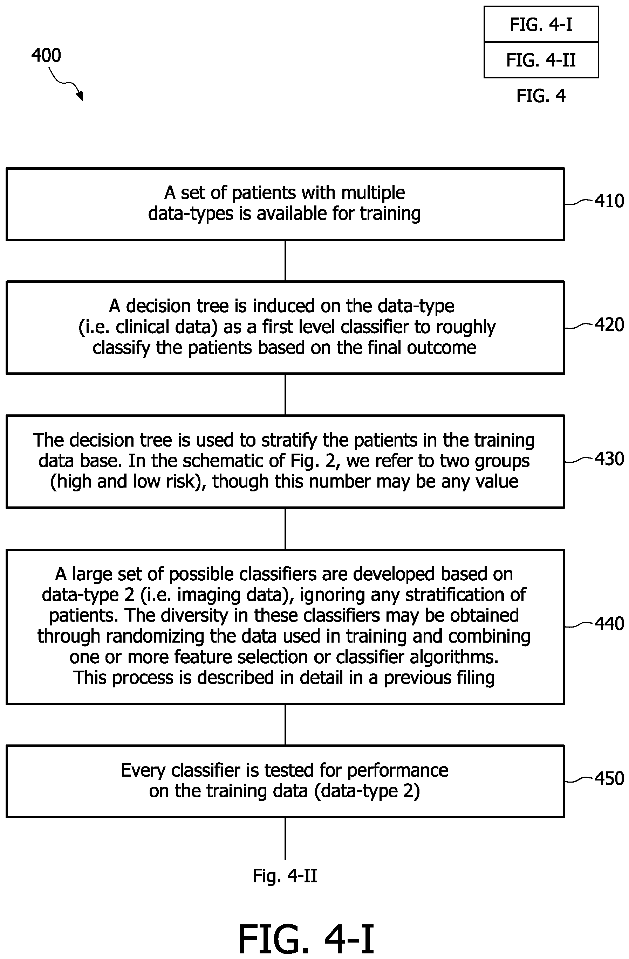

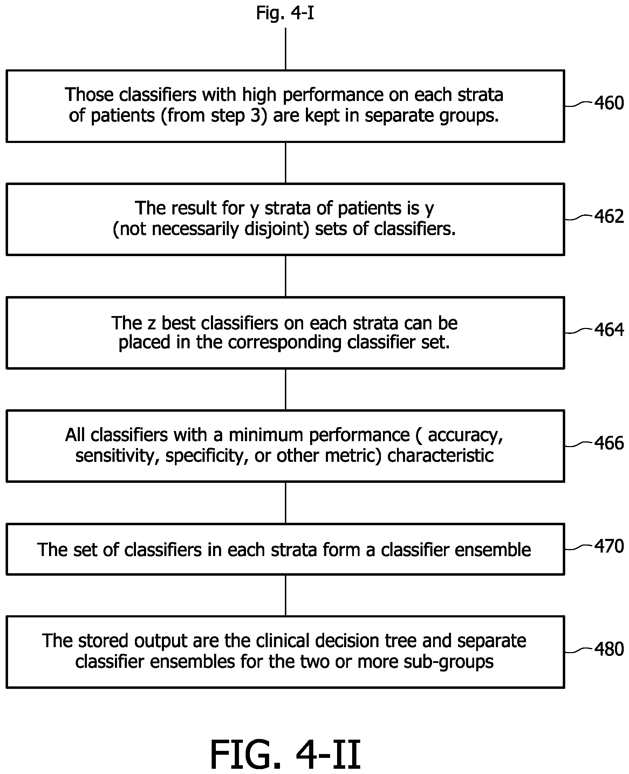

With reference to FIGURE 4, a second classifier selection 400 (Approach II) is presented based on selecting out one or more classifiers that are found to perform well for different sub-groups of patients. In a first step 410, a set of patients with multiple data-types is made available for training. Then, in step 420, a decision tree is induced on the data-type 1 (i.e. clinical data) as a first level classifier to roughly classify the patients based on the final outcome. The decision tree is used to stratify the patients in the training data base in step 430. In the Figure 4 method 400, we refer to these two groups of patient outcomes as high risk and low risk, though this number may be any value. A large set of possible classifiers are developed based on data-type 2 (i.e. clinical data) in st 440, ignoring any stratification of patients. The diversity in these classifiers can be obtained through randomizing the data used in training and combining one or more feature selection or classifier algorithms. Every classifier is tested in step 450 for performance on the training data (data-type 2).

In step 460, those classifiers with high performance on each strata of patients are kept in separate groups. The result 462 for y strata of patients is y, but not necessarily disjoint, sets of classifiers. Either the z best classifiers 464 on each strata can be placed in the corresponding classifier set, or all classifiers 466 with a minimum performance based on accuracy, sensitivity, specificity, or other metric characteristics. The set of classifiers in each strata form a classifier ensemble in step 470. In step 480, the clinical decision tree and separate classifier ensembles for the two or more sub-groups are stored as output.

A classifier is a categorization of a patient based on final outcome. An ensemble is a group of classifiers which are ranked based on ability to predict. Together the classifiers in an ensemble are able to predict better and more accurately than are the individual classifiers.

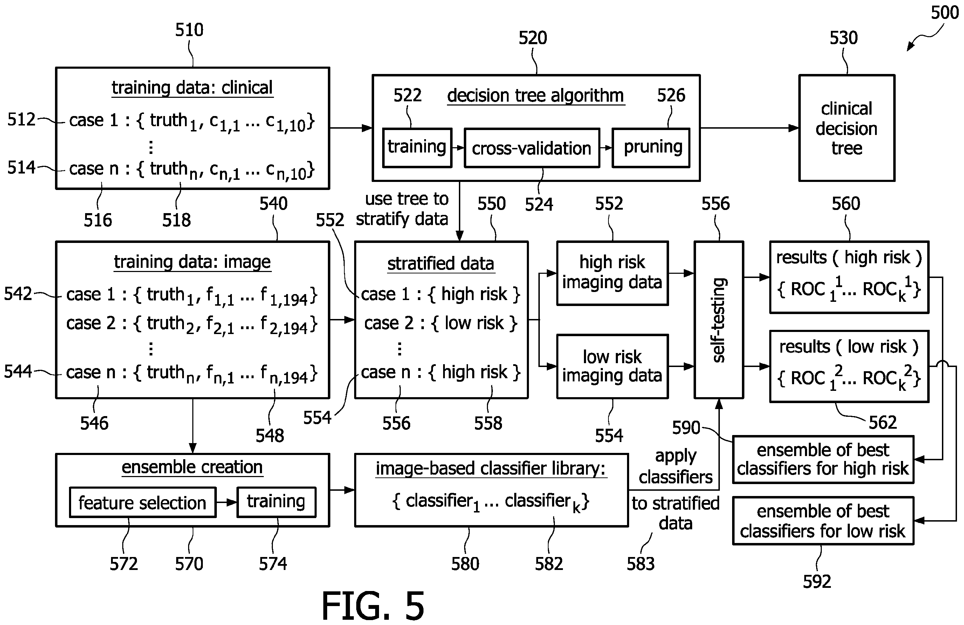

With reference to FIGURE 5, a wide variety of image-based classifiers are developed and then use the clinical data to decide which classifiers to use for different clinical 'risk' groups. The classifiers thus created are subsequently used for the computer-aided diagnosis of new, previously unseen patients 500. The method in which these classifiers are applied closely parallels the method shown in Figure 3.

Clinical data 510 is a collection of cases beginning with a first case 512 and proceeding to a given Nth number of cases 514, with each individual case representing a particular patient. Each case contains a name or identifier 516 of a patient and a series of attributes 518 gathered about the patient. The attributes include, but are not limited to, smoking, and exercise, or physical attributes such as but not limited to height and weight. These attributes also necessarily include the truth associated with the diagnosis in question, such as but not limited to whether the patient has cancer. These are accessed by the decision tree algorithm 520, which itself includes modules for training 522 for the creation of new decision tree branches, cross validation 524 for checking of branches, and pruning 526 for removing branches that are no longer relevant. The decision tree algorithm 520 is used to produces the clinical decision tree 530.

The training data for images 540 includes a series of cases beginning with a first case 542 and proceeding to an Nth case 544, with each individual case representing a particular patient. The cases 542, 544 represent the same patients as cases 512, 514. Each case contains a name or identifier 546 of a patient and a series of attributes 548 gathered about the patient and the medical images of the patient. The attributes necessarily include the truth associated with the diagnosis in question, such as but not limited to whether the patient has cancer. The attributes further include but are not limited to descriptive features of the images and regions of the images, such as but not limited to descriptors of contrast, texture, shape, intensity, and variations of intensity. These cases are used in combination with the decision tree algorithm 520 to create stratified data 550.

The stratified data 550 is a series of at least one case 552 to N cases 554 generated to determine if an individual case presents a high risk 556 or a low risk 558 of possessing a given illness or condition based on whether the probability of a person with a specific health background is likely to have or not have a given illness or condition, i.e. based on the information contained within the clinical data 510. A person with a high likelihood is classified as high risk 552 imaging data, while a person with a low likelihood of such an illness would be classified as low risk 554.

The image training data 540 is also sent to an ensemble module 570, comprised of a feature 572 selection part and a training 574 part. This ensemble creation creates and stores an image-based classifier library 580 comprised of a plurality of classifiers 582 which are able to associate cases 546 and their imaging attributes 548 with the appropriate diagnosis. These classifiers 582 are then applied 583 to the self testing data module 556. Both high risk 552 and low risk 554 persons would then be analyzed by self-testing 556.

Subsequently, a high risk result is a Receiver Operating Characteristic curve (ROC) processor 560. The ensemble of best classifiers for high risk are recorded in a high risk classifier area 590. Similarly, a low risk result would be sent to the low risk result ROC 562. The ensemble of best classifiers for low risk are recorded in a low risk classifier area 592.

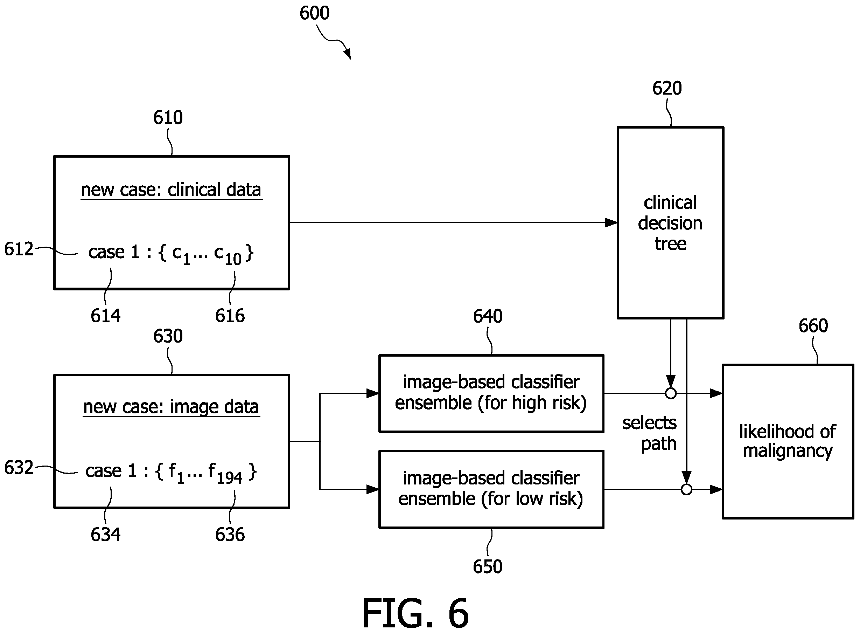

FIGURE 6 shows a schematic of how the classifier selection system would operate on new, unknown cases 600. A new case clinical data 610 module is comprised of at least one new case 612, which is made up of a case name 614 and a series of elements 616. This case is sent to a clinical decision tree 620 similar to the clinical decision tree 330 and 530 of Figs 3 and 5 respectively. One of two alternate paths is selected.

A new case image data 630 module is comprised of at least one new case 632, which is made up of a case name 634 and a series of elements 636. This at least one new case represents the same persons as is represented in the new case clinical data module 610. This case is sent to be classified by two alternate paths. In one path, the image based classifier ensemble for high risk 640 is used. This high risk classifier ensemble 640 is similar to the previously described modules 390 and 590. In a second path, the image based classifier ensemble for low risk 650 is used. This low risk classifier ensemble 650 is similar to the previously described modules 392 and 592. The result of the clinical decision tree is the use of paths to select which path is activated. The active path allows the result of one of the two image-based classifier ensemble results, (either the high risk result or low risk result), to be stored in the likelihood of malignancy module 660.

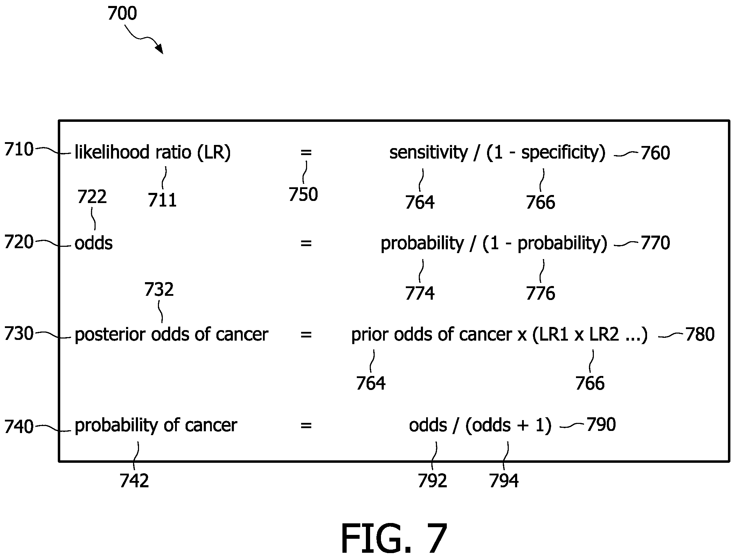

With reference to FIGURE 7, a third approach to splitting the CADx problem into parts is presented through the method of Bayesian analysis 700. Here, a summary of the key relevant equations of Bayesian analysis is used to analyze risk factors. The likelihood ratio of an affliction 710, abbreviated LR 711 is equal 750 to the formula 760 of sensitivity 764 divided by one minus the specificity 766. The odds 720 of occurrence 722 is equal 750 to the formula 770 of probability 774 divided by the value 776 of one minus the same probability 774. The posterior 730 odds of an illness 732 such as but not limited to cancer is equal 750 to the formula 780 of prior odds of cancer 764 times a succession of likelihood ratios 766 calculated in a manner similar to the likelihood ration 711. The probability of an illness 740, such as but not limited to the probability of cancer 742, is equal 750 to the formula 790 of odds 792 divided by the odds 792 plus one 794, where the odds 792 are calculated in a manner similar to the previously calculated odds 722, 732.

In this approach to enabling the present application, a CADx system based on the image features will be constructed. This image-based system will be used to first assign a likelihood of malignancy to an unknown case. This image-based CADx output will serve as a prior probability. This probability will be modulated based on Bayesian analysis of the clinical features. As described earlier, tests will be performed to see if the Bayesian modification of the probabilities affects the outcome of the final calculation. The user will be prompted for the clinical information only if it is deemed necessary by the comparison calculation.

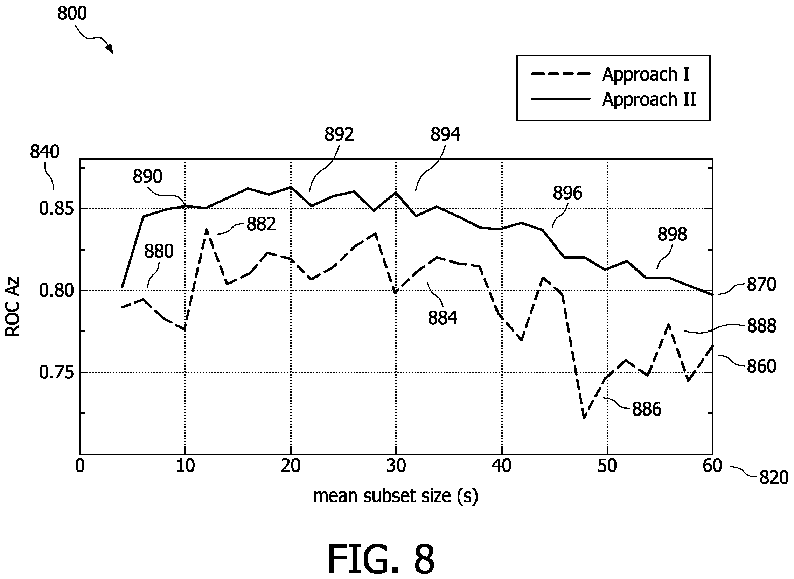

With reference to FIGURE 8, a proof-of-concept of the two classifier selection systems 300, 500 in a lung CADx application 800 is presented. Receiver Operating Characteristic curve (ROC) comprises a graphical plot for a binary classifier system formed by plotting 1 minus specificity on an X-axis and sensitivity on a Y-axis. The areas under this plotted curve is Az and is an index of accuracy. A value of 1.0 represents a perfect accurate test, a value of .9 to 1 represents an excellent test, a value of .8 to .9 represents a good test, a value of .7 to .8 represents a fair test, a value of .6 to .7 represents a poor test and a value below .6 represents a failing test. ROC Az represents the area under a ROC curve presenting these values.

Proof-of-concept tests have been performed using a pulmonary nodule data set. Classification was performed using a random subspace ensemble of linear discriminant classifiers.

A mean subset size is displayed on the X-axis increasing in size to a maximum value 820 of 60. The Y-axis contains the value ROC Az which increases to a maximum value 840 of approximately 0.9. The graph presents two approaches. In a first 860 Approach I derived in the manner of 300, as the subset size increases, the value of ROC Az steadily increases 880, reaches a peak 882, stabilizes 884, begins to fall 886 dramatically, and finishes above the lowest value 888. In a second 870 Approach II derived in the manner of 500, as subset size increases, the value of ROC Az steadily increases 890, stabilizes 892, reaches a peak 894, falls steadily 896, and finishes at its lowest value 898. Generally, the value of ROC Az increases for both Approaches I and II as mean subset size increases until the subset size reaches 30. Then the ROC Az begins to decrease as the subset size decreases. Approach II 870 is shown to be more accurate than Approach I 860. The Az to subset-size relationship is consistent with previously published results using conventional classifier ensemble methods. Therefore, we believe that the methods described herein can match the diagnostic accuracy of state-of-the-art CADx systems, while yielding the benefits of improved workflow and interface that is well-suited for clinical application.

Initial tests were further performed to demonstrate the appropriateness of the proposed approach 700. Leave-one-out CADx results without clinical features were combined with patient age information. A random subspace ensemble of linear discriminant classifiers was used to create the image-based classifier, resulting in an Az of 0.861. Combining this with age using Bayesian statistics results in an Az of 0.877. These results demonstrate the feasibility and potential for this Bayesian approach to data fusion.

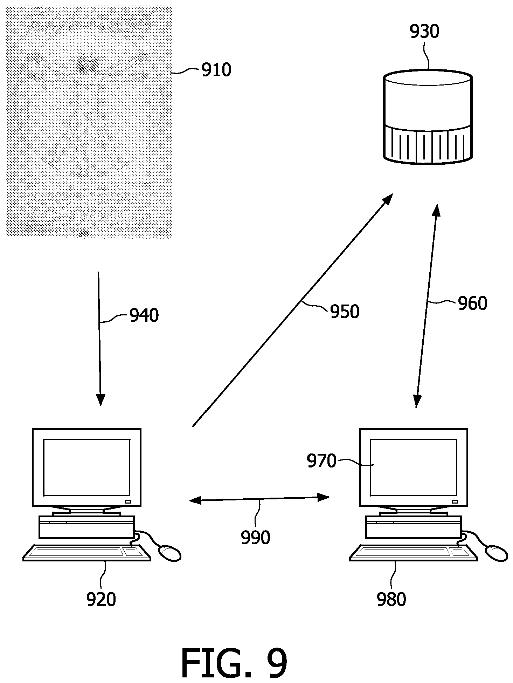

With reference to FIGURE 9, the system employs a medical image 910 that is input into a computer operable system 920 for processing. A decision engine executed on computer operable system 920 accesses a computer-based classifier system from a computer-aided diagnosis database 930. The classifier system is executed on the computer operable system 920 to compute a partial diagnosis based on the image data 910 and further compute potential complete diagnoses based on possible clinical data. The decision engine decides whether additional clinical data is required based on these diagnoses. If required, the request for additional clinical data is sent to interface engine 980 with display terminal 970 which queries the operator for additional information. If available, this additional information is then sent to the decision engine to compute a final diagnosis. This diagnosis is then sent to the computer display terminal 970. Alternatively, if the operator is unable to provide the additional information or if the decision engine decides that additional data is not required, then the partial results or the possible diagnoses computed by the decision engine can be displayed on the computer display terminal 970. The results of the computations are further stored in decision database 930. Communication may occur between the decision engine of the computer operable system 920 and the interface engine 980. Alternately, both the decision engine and the interface engine 980 may exist in the same computer apparatus.

Key applications within healthcare include image-based clinical decision support systems, in particular computer-aided diagnosis systems and clinical decision support (CDS) systems for therapy which may be integrated within medical imaging systems, imaging workstations, patient monitoring systems, and healthcare informatics. Specific image-based computer-aided diagnosis and therapy CDS systems include but are not limited to those for lung cancer, breast cancer, colon cancer, prostate cancer, based on CT, MRI, ultrasound, PET, or SPECT. Integration may involve using the present application in radiology workstations (e.g. PMW, Philips Extended Brilliance ™ Workstation) or PACS (e.g. iSite™).

The present application has been described with reference to the preferred embodiments. Modifications and alterations may occur to others upon reading and understanding the preceding detailed description. It is intended that the present application be construed as including all such modifications and alterations insofar as they come within the scope of the appended claims or the equivalents thereof.

## Claims

1. A system for providing computer-aided analysis of medical images comprising:
an image processor (910) for processing medical image data (940);
a decision engine (920) for generating a diagnosis based on the processed medical image data alone and further computing possible diagnostic outcomes based on different proposed possible values for clinical data;
a database (930) of prior diagnoses, their accompanying probabilities (960), and classifier algorithms for assessing probability of an illness given image data alone, image data with incomplete clinical data, and/or image data with clinical data;
an interface engine (980) for requesting and entering clinical data; and
a display terminal (970) for displaying the results of the computer-aided analysis,
wherein the system is further adapted to develop ensembles of classifiers for use in the computer aided analysis, the developing comprising
making available a set of patient cases, each with multiple data types, as a training database for training,
inducing a decision tree on the clinical data as a first level classifier to roughly classify the patients based on a final outcome (220),
using the decision tree to stratify the patients in the training data base into high risk or low risk of having a particular illness,
developing a set of possible classifiers based on the clinical data, ignoring any stratification of the patients,
performing a test for each of the classifiers (450) for performance on the training data, the classifiers with high performance on each strata of patients being kept in separate groups such that the result for y strata of the patients includes y sets of the classifiers,
placing the z best performing classifiers on each strata or all classifiers (460) with a minimum required performance of accuracy, sensitivity, or specificity, in the corresponding classifier set, the set of classifiers for each strata forming a classifier ensemble, and
storing the clinical decision tree and separate classifier ensembles as output.

2. The system according to claim 1, wherein the decision engine (920) displays an average diagnosis on the display terminal (970) and determines (160) what additional data, such as clinical data, is needed to make a definite diagnosis (660).

3. The system according to claim 1, wherein clinical data comprises at least one of medical history, health history, family history, physical measurements, and demographic data.

4. The system according to claim 1, wherein at least one of the image data or the clinical data is used to stratify data as being high risk or low risk for a specific illness (350).

5. The system according to claim 1, wherein the case database (930) is used to at least one of quantify risk factors, create an image-based classifier library, and derive an ensemble.

6. The system according to claim 1, wherein the decision engine (920) is arranged for:
determining the probability of an illness based on available image data and available clinical data;
re-determining the probability based with a range of potential values for unavailable clinical data;
comparing the probabilities with available data and the available data plus potential unavailable data;
estimating a likelihood of an illness based on the evaluation of the medical image data;
estimating the likelihoods of the specific illness based on the medical image data plus different values of clinical data; and
comparing the estimated likelihood to determine which unavailable data would significantly affect the estimated likelihood.

7. A method of providing computer-aided analysis of medical images, comprising:
processing medical image data (940);
generating a diagnosis based on the processed medical image data alone and further computing possible diagnostic outcomes based on different proposed possible values for clinical data;
assessing probability of an illness given image data alone, image data with incomplete clinical data, and/or image data with clinical data based on use of a database (930) of prior diagnoses, their accompanying probabilities (960), and classifier algorithms;
requesting and entering clinical data; and
displaying the results of the computer-aided analysis,
the method further comprising developing ensembles of classifiers for use in the computer aided analysis, the developing comprising
making available a set of patient cases, each with multiple data types, as a training database for training,
inducing a decision tree on the clinical data as a first level classifier to roughly classify the patients based on a final outcome (220),
using the decision tree to stratify the patients in the training data base into high risk or low risk of having a particular illness,
developing a set of possible classifiers based on the clinical data, ignoring any stratification of the patients,
performing a test for each of the classifiers (450) for performance on the training data, the classifiers with high performance on each strata of patients being kept in separate groups such that the result for y strata of the patients includes y sets of the classifiers,
placing the z best performing classifiers on each strata or all classifiers (460) with a minimum required performance of accuracy, sensitivity or specificity, in the corresponding classifier set, the set of classifiers for each strata forming a classifier ensemble, and
storing the clinical decision tree and separate classifier ensembles as output.

8. A computer-aided diagnosis (CADx) system comprising:
a processor (920) programmed to perform the method according to claim 7; and
a display (970) which displays the results of the computer-aided analysis.

9. A computer programmable medium comprising a computer program which when loaded on a computer controls the computer to perform the method according to claim 7.

## Patentansprüche

1. System zum Bereitstellen einer computergestützten Analyse von medizinischen Bildern, umfassend:
einen Bildprozessor (910) zum Verarbeiten von medizinischen Bilddaten (940);
eine Entscheidungsmaschine (920) zum Erstellen einer Diagnose allein aufgrund der verarbeiteten medizinischen Bilddaten und zur weiteren Berechnung möglicher Diagnoseergebnisse aufgrund von verschiedenen vorgeschlagenen möglichen Werten für klinische Daten;
eine Datenbank (930) mit früheren Diagnosen, den jeweils dazugehörigen Wahrscheinlichkeiten (960) sowie Klassifizierungsalgorithmen zur Beurteilung der Wahrscheinlichkeit einer Erkrankung allein aufgrund von Bilddaten, Bilddaten mit unvollständigen klinischen Daten und/oder Bilddaten mit klinischen Daten;
eine Schnittstellenmaschine (980) zum Anfordern und Eingeben von klinischen Daten; und
ein Anzeigeendgerät (970) zum Anzeigen der Ergebnisse der computergestützten Analyse,
wobei das System weiter angepasst ist, um Ensembles von Klassifikatoren zur Verwendung bei der computergestützten Analyse zu entwickeln, die Entwicklung umfassend
Bereitstellung einer Reihe von Patientenfällen mit jeweils mehreren Datentypen als Schulungsdatenbank für die Schulung,
Erzeugen eines Entscheidungsbaums aufgrund der klinischen Daten als Klassifikator der ersten Ebene, um die Patienten aufgrund eines Endergebnisses grob zu klassifizieren (220),
Verwendung des Entscheidungsbaums, um die Patienten in der Schulungsdatenbank nach einem hohen oder geringen Risiko für eine bestimmte Erkrankung zu stratifizieren,
Entwicklung einer Reihe möglicher Klassifikatoren aufgrund der klinischen Daten, wobei jegliche Stratifizierung der Patienten ignoriert wird,
Durchführen eines Tests für jeden der Klassifikatoren (450) auf Aussagekraft in Bezug auf die Schulungsdaten, wobei die Klassifikatoren mit hoher Aussagekraft über jedes Stratum von Patienten in separaten Gruppen geführt werden, sodass das Ergebnis für y Strata der Patienten y Sätze der Klassifikatoren beinhaltet,
Platzieren der z aussagekräftigsten Klassifikatoren auf jedem Stratum oder aller Klassifikatoren (460) mit einer erforderlichen Mindestaussagekraft in punkto Genauigkeit, Empfindlichkeit oder Spezifität in dem entsprechenden Klassifikatoren-Satz, wobei der Satz von Klassifikatoren für jedes Stratum ein Klassifikatoren-Ensemble bildet, und
Speichern des klinischen Entscheidungsbaums und separater Klassifikatoren-Ensembles als Ausgabe.

2. System nach Anspruch 1, wobei die Entscheidungsmaschine (920) eine durchschnittliche Diagnose auf dem Anzeigeendgerät (970) anzeigt und bestimmt (160), welche zusätzlichen Daten, wie beispielsweise klinische Daten, benötigt werden, um eine endgültige Diagnose (660) zu stellen.

3. System nach Anspruch 1, wobei klinische Daten mindestens eines von Anamnese, Krankengeschichte, Familiengeschichte, physikalischen Messwerten und demographischen Daten umfassen.

4. System nach Anspruch 1, wobei mindestens eine der Bilddaten oder der klinischen Daten verwendet wird, um Daten als hohes oder geringes Risiko für eine bestimmte Erkrankung (350) zu stratifizieren.

5. System nach Anspruch 1, wobei die Falldatenbank (930) verwendet wird, um mindestens einen der Risikofaktoren zu quantifizieren, eine bildbasierte Klassifikatorenbibliothek zu erstellen und ein Ensemble abzuleiten.

6. System nach Anspruch 1, wobei die Entscheidungsmaschine (920) eingerichtet ist:
zum Bestimmen der Wahrscheinlichkeit einer Erkrankung aufgrund von verfügbaren Bilddaten und verfügbaren klinischen Daten;
zur Neubestimmung der Wahrscheinlichkeit aufgrund einer Reihe von potenziellen Werten für nicht verfügbare klinische Daten;
zum Vergleich der Wahrscheinlichkeiten mit verfügbaren Daten sowie den verfügbaren Daten plus potenziell nicht verfügbaren Daten;
zum Schätzen der Wahrscheinlichkeit einer Erkrankung aufgrund der Auswertung der medizinischen Bilddaten;
zum Schätzen der Wahrscheinlichkeit der spezifischen Erkrankung aufgrund der medizinischen Bilddaten zuzüglich verschiedener Werte klinischer Daten; und
zum Vergleichen der geschätzten Wahrscheinlichkeit, um zu ermitteln, welche nicht verfügbaren Daten die geschätzte Wahrscheinlichkeit erheblich beeinflussen würden.

7. Verfahren zum Bereitstellen einer computergestützten Analyse von medizinischen Bildern, umfassend:
Verarbeitung von medizinischen Bilddaten (940);
Erstellen einer Diagnose allein aufgrund der verarbeiteten medizinischen Bilddaten und weitere Berechnung möglicher Diagnoseergebnisse aufgrund verschiedener vorgeschlagener möglicher Werte für klinische Daten;
Beurteilen der Wahrscheinlichkeit einer Erkrankung allein durch Bilddaten, Bilddaten mit unvollständigen klinischen Daten und/oder Bilddaten mit klinischen Daten, basierend auf der Verwendung einer Datenbank (930) mit früheren Diagnosen, den jeweils dazugehörigen Wahrscheinlichkeiten (960) und den Klassifizierungsalgorithmen;
Anforderung und Eingabe von klinischen Daten; und
Anzeigen der Ergebnisse der computergestützten Analyse,
wobei das Verfahren weiter die Entwicklung von Ensembles von Klassifikatoren zur Verwendung bei der computergestützten Analyse umfasst, die Entwicklung umfassend,
Bereitstellung einer Reihe von Patientenfällen mit jeweils mehreren Datentypen als Schulungsdatenbank für die Schulung,
Erzeugen eines Entscheidungsbaums aufgrund der klinischen Daten als Klassifikator der ersten Ebene, um die Patienten basierend auf einem Endergebnis grob zu klassifizieren (220),
Verwendung des Entscheidungsbaums, um die Patienten in der Schulungsbank nach einem hohen oder geringen Risiko für eine bestimmte Krankheit zu stratifizieren,
Entwicklung einer Reihe möglicher Klassifikatoren aufgrund der klinischen Daten, wobei jegliche Stratifizierung der Patienten ignoriert wird,
Durchführen eines Tests für jeden der Klassifikatoren (450) auf Aussagekraft in Bezug auf die Schulungsdaten, wobei die Klassifikatoren mit hoher Aussagekraft über jedes Stratum von Patienten in separaten Gruppen geführt werden, sodass das Ergebnis für y Strata der Patienten y Sätze der Klassifikatoren beinhaltet,
Platzieren der z aussagekräftigsten Klassifikatoren auf jedem Stratum oder aller Klassifikatoren (460) mit einer erforderlichen Mindestaussagekraft in punkto Genauigkeit, Empfindlichkeit oder Spezifität in dem entsprechenden Klassifikatoren-Satz, wobei der Satz von Klassifikatoren für jedes Stratum ein Klassifikatoren-Ensemble bildet, und
Speichern des klinischen Entscheidungsbaums und separater Klassifikatoren-Ensembles als Ausgabe.

8. Computergestütztes Diagnosesystem (CADx), umfassend:
einen Prozessor (920), der programmiert ist, um das Verfahren nach Anspruch 7 durchzuführen; und
eine Anzeige (970), die die Ergebnisse der computergestützten Analyse anzeigt.

9. Computerprogrammierbares Medium, umfassend ein Computerprogramm, das, wenn es auf einen Computer geladen wird, den Computer steuert, um das Verfahren nach Anspruch 7 durchzuführen.

## Revendications

1. Système pour fournir une analyse assistée par ordinateur d'images médicales comprenant :
un processeur d'image (910) pour traiter des données d'image médicale (940) ;
un moteur de décision (920) pour générer un diagnostic sur la base des seules données d'image médicale traitées et calculer en outre des résultats de diagnostic possibles sur la base de différentes valeurs possibles proposées pour des données cliniques ;
une base de données (930) de diagnostics antérieurs, leurs probabilités associées (960), et des algorithmes classificateurs pour évaluer la probabilité d'une maladie à partir des seules données d'image, de données d'image avec des données cliniques incomplètes, et/ou de données d'image avec des données cliniques ;
un moteur d'interface (980) pour demander et entrer des données cliniques ; et
un terminal d'affichage (970) pour afficher les résultats de l'analyse assistée par ordinateur,
dans lequel le système est adapté en outre pour développer des ensembles de classificateurs pour une utilisation dans l'analyse assistée par ordinateur, le développement comprenant
le fait de rendre disponible un jeu de cas de patients, chacun avec de multiples types de données, en tant que base de données d'apprentissage à des fins d'apprentissage,
l'induction d'un arbre de décision sur les données cliniques en tant que classificateur de premier niveau pour classer grossièrement les patients sur la base d'un résultat final (220),
l'utilisation de l'arbre de décision pour stratifier les patients dans la base de données d'apprentissage en risque élevé ou risque bas d'avoir une maladie particulière,
le développement d'un jeu de classificateurs possibles sur la base des données cliniques, en ignorant n'importe quelle stratification des patients,
la mise en oeuvre d'un test de performance pour chacun des classificateurs (450) sur les données d'apprentissage, les classificateurs ayant une performance élevée sur chaque strate de patients étant conservés dans des groupes séparés de façon que le résultat pour y strates des patients inclut y jeux des classificateurs,
le placement des z classificateurs les plus performants sur chaque strate ou de tous les classificateurs (460) ayant une performance minimale requise de précision, de sensibilité, ou de spécificité, dans le jeu de classificateurs correspondant, le jeu de classificateurs pour chaque strate formant un ensemble de classificateurs, et
le stockage de l'arbre de décision clinique et des ensembles de classificateurs séparés en tant que sortie.

2. Système selon la revendication 1, dans lequel le moteur de décision (920) affiche un diagnostic moyen sur le terminal d'affichage (970) et détermine (160) quelles sont les données supplémentaires, telles que des données cliniques, nécessaires pour faire un diagnostic définitif (660).

3. Système selon la revendication 1, dans lequel les données cliniques comprennent au moins l'un parmi des antécédents médicaux, un historique de santé, des antécédents familiaux, des mesures physiques, et des données démographiques.

4. Système selon la revendication 1, dans lequel au moins l'un parmi les données d'image ou les données cliniques est utilisé pour stratifier les données comme étant à risque élevé ou à risque bas pour une maladie spécifique (350).

5. Système selon la revendication 1, dans lequel la base de données de cas (930) est utilisée pour au moins l'un parmi quantifier les facteurs de risque, créer une banque de classificateurs à base d'images, et dériver un ensemble.

6. Système selon la revendication 1, dans lequel le moteur de décision (920) est agencé pour :
déterminer la probabilité d'une maladie sur la base de données d'image disponibles et de données cliniques disponibles ;
déterminer de nouveau la probabilité sur la base d'une plage de valeurs potentielles pour des données cliniques non disponibles ;
comparer les probabilités aux données disponibles et aux données disponibles plus aux données non disponibles potentielles ;
estimer une vraisemblance d'une maladie sur la base de l'évaluation des données d'image médicale ;
estimer les vraisemblances de la maladie spécifique sur la base des données d'image médicale plus de différentes valeurs de données cliniques ; et
comparer la vraisemblance estimée pour déterminer les données non disponibles qui affecteraient significativement la vraisemblance estimée.

7. Procédé de fourniture d'une analyse assistée par ordinateur d'images médicales, comprenant :
le traitement de données d'image médicale (940) ;
la génération d'un diagnostic sur la base des seules données d'image médicale traitées et en outre le calcul de résultats de diagnostic possibles sur la base de différentes valeurs possibles proposées pour des données cliniques ;
l'évaluation de la probabilité d'une maladie à partir des seules données d'image, de données d'image avec des données cliniques incomplètes, et/ou de données d'image avec des données cliniques par l'utilisation d'une base de données (930) de diagnostics antérieurs, de leurs probabilités associées (960), et d'algorithmes classificateurs ;
la demande et l'entrée de données cliniques ; et
l'affichage des résultats de l'analyse assistée par ordinateur,
le procédé comprenant en outre le développement d'ensembles de classificateurs pour une utilisation dans l'analyse assistée par ordinateur, le développement comprenant
le fait de rendre disponible un jeu de cas de patients, chacun avec de multiples types de données, en tant que base de données d'apprentissage à des fins d'apprentissage,
l'induction d'un arbre de décision sur les données cliniques en tant que classificateur de premier niveau pour classer grossièrement les patients sur la base d'un résultat final (220),
l'utilisation de l'arbre de décision pour stratifier les patients dans la base de données d'apprentissage en risque élevé ou risque bas d'avoir une maladie particulière,
le développement d'un jeu de classificateurs possibles sur la base des données cliniques, en ignorant n'importe quelle stratification des patients,
la mise en oeuvre d'un test de performance pour chacun des classificateurs (450) sur les données d'apprentissage, les classificateurs ayant une performance élevée sur chaque strate de patients étant conservés dans des groupes séparés de façon que le résultat pour y strates des patients inclut y jeux des classificateurs,
le placement des z classificateurs les plus performants sur chaque strate ou de tous les classificateurs (460) ayant une performance minimale requise de précision, de sensibilité, ou de spécificité, dans le jeu de classificateurs correspondant, le jeu de classificateurs pour chaque strate formant un ensemble de classificateurs, et
le stockage de l'arbre de décision clinique et des ensembles de classificateurs séparés en tant que sortie.

8. Système de diagnostic assisté par ordinateur (CADx) comprenant :
un processeur (920) programmé pour mettre en oeuvre le procédé selon la revendication 7; et
un affichage (970) qui affiche les résultats de l'analyse assistée par ordinateur.

9. Support programmable par ordinateur comprenant un programme informatique qui, quand il est chargé sur un ordinateur, commande l'ordinateur pour mettre en oeuvre le procédé selon la revendication 7.
